# EUROPEAN PATENT APPLICATION

(11) **EP 2 241 276 A1**
(43) Date of publication of application: **20.10.2010**
(21) Application number: 10250755.5
(22) Date of filing: 12.04.2010
(51) Int. Cl.: A61B 17/34, A61B 17/00, A61B 18/00

(54) **Bendable veress needle assembly**

(30) Priority: 13.04.2009 US 168664 P; 16.03.2010 US 724515
(71) Applicant: Tyco Healthcare Group LP, North Haven, CT 06473 (US)
(72) Inventor: Davis, Michael, Middletown, CT 06457 (US); Hathaway, Peter, Lebanon, CT 06249 (US)
(74) Representative: Soames, Candida Jane

(57) **Abstract**

A bendable veress needle is disclosed that has a shaft with a bendable region located near its distal end. The distal end of the bendable region includes a tissue penetrating tip. A stylet may be inserted through the bendable veress needle. A housing having an insufflation port is located at a proximal end of the bendable veress needle. A light source or imaging system may be coupled to the bendable veress needle.

## Description

### CROSS REFERENCE TO RELATED APPLICATION

The present application claims the benefit of and priority to U.S. Provisional Application Serial No. 61/168,664 filed on April 13, 2009, the entire contents of which are incorporated herein by reference.

### BACKGROUND

### 1. Technical field

The present disclosure relates to veress needles. More particularly, the present disclosure relates to bendable veress needle assemblies used for surgical incisions.

### 2. Background of Related Art

During various surgical procedures it is often desirable to access areas within a patient's body in a relatively non-invasive manner. In laparoscopic and/or endoscopic surgeries, a small incision is formed in the body to allow passage of various surgical instrumentation. Often these incisions are formed using a hollow pointed needle also referred to as a veress needle.

In some specific surgeries, such as, for example, hernia repair surgery, a small incision is made through the abdominal wall to access tissue within the abdominal cavity. Some such procedures require insufflation of the abdominal cavity to provide an operative space. This is typically accomplished with the insertion of a cannula through the incision. Additional incisions may be made through the abdominal wall to accommodate additional cannulas and surgical instrumentation.

Occasionally, the surgical instruments used to form the incision result in tears or nonuniform areas around the incision making it difficult to seal about the incision for proper insufflation. Additionally, there is a risk of over insertion resulting in penetration and damage to underlying anatomical structure.

### SUMMARY

The presently disclosed bendable veress needle includes a shaft with a bendable distal region. A piercing tip is located at a distal end of the shaft. A stylet may be inserted through the shaft. The stylet may be configured for transmitting light from a light source external to the patient and/or for viewing a region of the body during or subsequent to insertion of the bendable veress needle through an incision in the patient's skin. A housing is located at a proximal end of the bendable veress needle. The housing may include an insufflation port for introducing insufflation fluids into a cavity through a bore of the bendable veress needle. The bendable veress needle may be biased towards a bent position such that, upon insertion of the bendable veress needle through an incision in the patient's skin, the distal end thereof assumes a bent position so as to minimize the likelihood that the distal tip will damage underlying anatomical structures.

In various embodiments, there is provided a veress needle that comprises a shaft having a bendable region, a piercing tip being located at a distal end of the shaft; and a housing located at a proximal end of the shaft, the housing including an insufflation port for introducing insufflation fluids through a bore of the shaft. A stylet may be insertable through the shaft. The stylet may be configured for transmitting light from a light source external to a patient into a body cavity of the patient. Also, the stylet may be coupleable to an imaging system for viewing a region of the body during or subsequent to insertion of the shaft through a patient's skin. The bendable region maybe located near a distal portion of the shaft. The bendable region may be biased towards a bent position. Upon insertion of the bendable region through a patient's skin, the distal region may assume a bent position so as to minimize the likelihood that the piercing tip at the distal end of the shaft will damage underlying anatomical structures. The distal region may be bendable by an operator. The veress needle may also include a control structure actuatable by the operator such that the distal region may be selectively bent by the operator. The bendable region may be formed from a shape memory alloy.

### BRIEF DESCRIPTION OF THE DRAWINGS

Various embodiments of the presently disclosed bendable veress needle assembly are disclosed herein with reference to the drawings, wherein:
Fig. 1 is a side, cross-sectional view of one embodiment of a bendable veress needle assembly inserted through tissue; and
Fig. 2 is a side view of a distal region of the bendable veress needle of Fig. 1.

### DETAILED DESCRIPTION

Embodiments of the presently disclosed bendable veress needle assembly will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

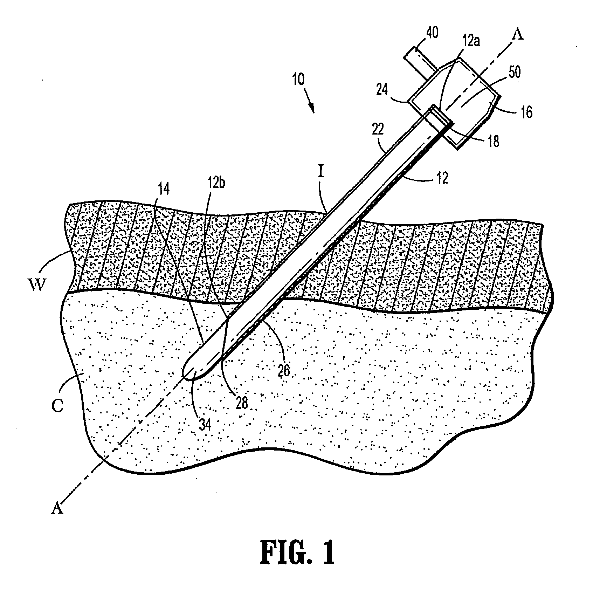

Referring initially to Fig. 1, a bendable veress needle assembly 10 includes a bendable veress needle 12 having a shaft 22. The shaft 22 includes an open proximal end 12a and an open distal end 12b. The open proximal and distal ends 12a, 12b define a bore through the shaft 22. A stylet 14 is introducible through the bore of the shaft 22 and includes a lens 34. The lens 34 transmits light therethrough allowing a light source (not shown) to be coupled to the stylet 14 for illuminating the operative site in a body cavity C. Alternatively, the stylet 14 may be coupled to a camera or other imaging system for viewing the operative site in the body cavity C. A tissue penetrating tip 28 is located at the distal end 12b of the shaft 22. Further still, a housing 16 is coupled to the proximal end 12a of the shaft 22. The housing includes an insufflation port 40 that allows the introduction of an insufflation fluid (e.g. gas or liquid) into the body cavity C.

The tissue penetrating tip 28 is formed at an angle relative to the shaft 22 and facilitates insertion through a patient's skin. As shown in Fig. 1, the shaft 22 is inserted into an incision I and penetrates through a wall W. The stylet 14 allows the clinician to introduce light into the cavity C, thereby illuminating the cavity and/or, with an optical imaging device coupled to the bendable veress needle 12, view the cavity C.

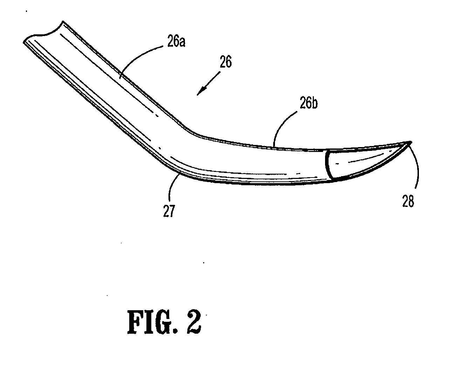

Referring additionally to Fig. 2, the shaft 22 includes a bendable region 26. The bendable region 26 includes a first portion 26a, a second portion 26b, and a coupling portion 27. In one embodiment, the coupling portion 27 is formed from a flexible or bendable material allowing relative flexion or bending between first portion 26a and second portion 26b. As configured, either before insertion, during insertion, or subsequent to insertion, the second portion 26b may be repositioned relative to the first portion 26a. This permits the clinician to control the insertion path of the shaft 22. As such, the clinician can reposition the second portion 26b relative to the first portion 26a.

By allowing relative movement between the first and second portions 26a, 26b, the shaft 22 may be inserted at an external location that is convenient for access, while allowing movement of the penetrating tip 28 to access an internal area that is distant from the entry point. Additionally, the inclusion of a bendable region 26 allows the clinician to use the stylet 14 for visualizing the cavity C during insertion of the shaft 22 and adjusting the insertion path to avoid damaging internal structures in the cavity C.

As discussed above, the bendable region 26 includes a first portion 26a, a second portion 26b, and a coupling portion 27. In one embodiment, the first portion 26a is formed of a rigid material that also forms the shaft 22, while the second portion 26b is formed of a bendable material. It is envisioned that the bendable material may or may not be a resilient material. In this configuration, the second portion 26b and the tissue penetrating tip 28 are repositionable with respect to the shaft 22 allowing the clinician to control the path of the bendable veress needle 10 through tissue.

Alternatively, the first and second portions 26a, 26b may be formed of a bendable or resilient material. In this embodiment, the bendable region 26 is formed from a different material than that used in constructing the shaft 22. By constructing both portions 26a, 26b of the bendable region 26 from a bendable or resilient material, greater flexibility is achieved. In yet a further embodiment, one or both of the first and second portions 26a, 26b may be formed from a shape memory alloy such as a nickel-titanium alloy or another suitable, biocompatible alloy. In either embodiment, the bendable region 26 is adapted for cooperating with the stylet 14. As such, the bendable veress needle 10 may be introduced through the patient's skin as a linear device and the bendable region 26 would conform to a predetermined or variable configuration of the stylet 14. This arrangement allows the clinician to insert the bendable veress needle 10 through tissue and select an appropriate stylet 14 having a geometric configuration suitable for the intended surgical procedure.

Furthermore, it is contemplated that one or both of the first and second portions 26a, 26b may include wires or other control structures that permit manipulation of the first and second portions 26a, 26b from the proximal end of the bendable veress needle 10. In this arrangement, the clinician is able to adjust the path of the bendable veress needle 10 as it is introduced through tissue. Also, the bendable veress needle may be biased towards a bent position such that, upon insertion of the bendable veress needle through an incision in the patient's skin, the distal end thereof assumes a bent position so as to minimize the likelihood that the distal tip will damage underlying anatomical structures.

It will be understood that various modifications may be made to the embodiments disclosed herein. Therefore, the above description should not be construed as limiting, but merely as exemplifications of particular embodiments. Those skilled in the art will envision other modifications within the scope and spirit of the claims appended hereto.

## Claims

1. A veress needle comprising:
a shaft having a bendable region, a piercing tip being located at a distal end of the shaft;
a housing located at a proximal end of the shaft, the housing including an insufflation port for introducing insufflation fluids through a bore of the shaft.

2. The veress needle of claim 1, further comprising a stylet insertable through the shaft.

3. The veress needle of claim 2, wherein the stylet is configured for transmitting light from a light source external to a patient into a body cavity of the patient.

4. The veress needle of any preceding claim, wherein the stylet is coupleable to an imaging system for viewing a region of the body during or subsequent to insertion of the shaft through a patient's skin.

5. The veress needle of any preceding claim, wherein the bendable region is located near a distal portion of the shaft

6. The veress needle of claim 5, wherein the bendable region is biased towards a bent position.

7. The veress needle of claim 6, wherein, upon insertion of the bendable region through a patient's skin, the distal region assumes a bent position so as to minimize the likelihood that the piercing tip at the distal end of the shaft will damage underlying anatomical structures.

8. The veress needle of any preceding clam, wherein the distal region is bendable by an operator.

9. The veress needle of claim 8, further comprising a control structure actuatable by the operator such that the distal region may be selectively bent by the operator.

10. The veress needle of claim 8, wherein the control structure includes wires actuatable by the operator to selectively bend the bendable region.

11. The veress needle of any preceding claim, wherein the bendable region is formed from a shape memory alloy
